# EUROPEAN PATENT APPLICATION

(11) **EP 2 949 396 A1**
(43) Date of publication of application: **02.12.2015**
(21) Application number: 15169380.1
(22) Date of filing: 27.05.2015
(51) Int. Cl.: B01L 7/00

(54) **SUBSTANCE AMPLIFICATION REACTION APPARATUS AND METHOD OF AMPLIFYING SUBSTANCE**

(30) Priority: 28.05.2014 JP 2014110427
(71) Applicant: Seiko Epson Corporation, Tokyo 163 (JP)
(72) Inventor: Togashi, Ken, Suwa-shi, Nagano 392-8502 (JP); Murayama, Toshiro, Suwa-shi, Nagano 392-8502 (JP)
(74) Representative: Hoffmann Eitle

(57) **Abstract**

A substance amplification reaction apparatus includes a mounting section mountable with a substance amplification reaction container (10) filled with reaction liquid (14) and liquid (15) that has specific gravity smaller than the specific gravity of the reaction liquid (14) and does not mix with the reaction liquid (14) and having a channel in which a first channel and a second channel are connected via a bent section and the reaction liquid (14) moves, a first to third heating sections (200, 300, 400) respectively capable of heating a first region (20), which is a region of an end portion on the first channel side of the channel, a second region (30), which is a region of the bent section, and a third region (40), which is a region at an end portion on the second channel side of the channel, and a driving mechanism configured to switch first arrangement, second arrangement, and third arrangement in this order. Each of the first to third arrangements is arrangement in which each of the first to third region (40)s is below the other regions.

## Description

### BACKGROUND

### 1. Technical Field

The present invention relates to a substance amplification reaction apparatus and a method of amplifying a substance.

### 2. Related Art

As a method of amplifying nucleic acid at high speed, there is known a nucleic acid amplification reaction apparatus that injects oil and a small amount of nucleic acid amplification reaction liquid into a nucleic acid amplification reaction container, maintains one end of the container at high temperature and maintains the other end at low temperature, and causes a heat cycle in the temperature of the nucleic acid amplification reaction liquid at high speed by rotating the container to alternately switch a state in which the one end is placed lower in the vertical direction to locate the reaction liquid in the high-temperature oil and a state in which the other end is placed lower in the vertical direction to locate the reaction liquid in the low-temperature oil (see, for example, JP-A-2012-115208 (Patent Literature 1)).

The nucleic acid amplification reaction apparatus described in Patent Literature 1 is based on two-step PCR. It is likely that deterioration in amplification efficiency under a specific reaction condition occurs. When a primer shorter than 20 nucleotides is used, amplification is difficult.

### SUMMARY

The object of the invention is to provide a substance amplification reaction apparatus for efficiently amplifying a substance, and an associated method of amplifying a substance.

An aspect of the invention is directed to a substance amplification reaction apparatus including: a mounting section mountable with a substance amplification reaction container filled with reaction liquid and liquid that has specific gravity smaller than the specific gravity of the reaction liquid and does not mix with the reaction liquid and having a channel in which the reaction liquid moves, the channel including a first channel and a second channel, the first channel and the second channel being connected via a bent section, and an axis of the first channel and an axis of the second channel forming an angle smaller than 90 degrees; a first heating section capable of heating a first region, which is a region of an end portion on the first channel side of the channel; a second heating section capable of heating a second region, which is a region of the bent section; a third heating section capable of heating a third region, which is a region at an end portion on the second channel side of the channel; a driving mechanism configured to switch first arrangement, second arrangement, and third arrangement in this order; and a control section configured to control the driving mechanism to maintain the first arrangement for a predetermined time, thereafter maintain the second arrangement for the predetermined time, and thereafter maintain the third arrangement for the predetermined time. The first arrangement is arrangement in which the first region is below the second region and the third region in a direction in which the gravity acts. The second arrangement is arrangement in which the second region is below the first region and the third region in the direction in which the gravity acts. The third arrangement is arrangement in which the third region is below the first region and the second region in the direction in which the gravity acts. The first heating section may include a first hole. The first region may be insertable into the first hole. The second heating section may include a second hole. The second region may be insertable into the second hole. The third heating section may include a third hole. The third region may be insertable into the third hole. The first to third holes may be the mounting section.

Another embodiment of the invention is directed to a method of amplifying a substance using a substance amplification reaction apparatus, the substance amplification reaction apparatus including: a mounting section mountable with a substance amplification reaction container filled with reaction liquid and liquid that has specific gravity smaller than the specific gravity of the reaction liquid and does not mix with the reaction liquid and having a channel in which the reaction liquid moves, the channel including a first channel and a second channel, the first channel and the second channel being connected via a bent section, and an axis of the first channel and an axis of the second channel forming an angle smaller than 90 degrees; a first heating section capable of heating a first region, which is a region of an end portion on the first channel side of the channel; a second heating section into which a second region, which is a region of the bent section, is insertable; and a third heating section capable of heating a third region, which is a region at an end portion on the second channel side of the channel. The method includes maintaining, for a predetermined time, first arrangement in which the first region is below the second region and the third region in a direction in which the gravity acts, thereafter, transitioning to second arrangement in which the second region is below the first region and the third region in the direction in which the gravity acts and maintaining the second arrangement for the predetermined time, and thereafter transitioning to third arrangement in which the third region is below the first region and the second region in the direction in which the gravity acts and maintaining the third arrangement for the predetermined time.

In both the aspects, the substance may be nucleic acid.

According to the aspects of the invention, it is possible to provide the substance amplification reaction apparatus for efficiently amplifying the substance.

### BRIEF DESCRIPTION OF THE DRAWINGS

The invention will be described with reference to the accompanying drawings, wherein like numbers reference like elements.
Fig. 1 is a schematic diagram of a substance reaction container and a heater in a substance amplification reaction apparatus according to an embodiment.
Figs. 2A to 2C are schematic diagrams showing a method of amplifying a substance using the substance amplification reaction apparatus according to the embodiment.
Fig. 3 is a flowchart for explaining a procedure of processing for amplifying a substance using the substance amplification reaction apparatus according to the embodiment.

### DESCRIPTION OF EXEMPLARY EMBODIMENTS

Preferred embodiments of the invention are explained below with reference to the drawings. Note that the embodiments explained below do not unduly limit contents of the invention described in the appended claims. Not all of components explained below are essential constituent elements of the invention.

### 1. Substance amplification reaction apparatus according to an embodiment

In an embodiment, a substance amplification reaction apparatus includes three heating sections on which a substance amplification reaction container is mountable and a driving mechanism capable of switching the arrangement of the heating sections.

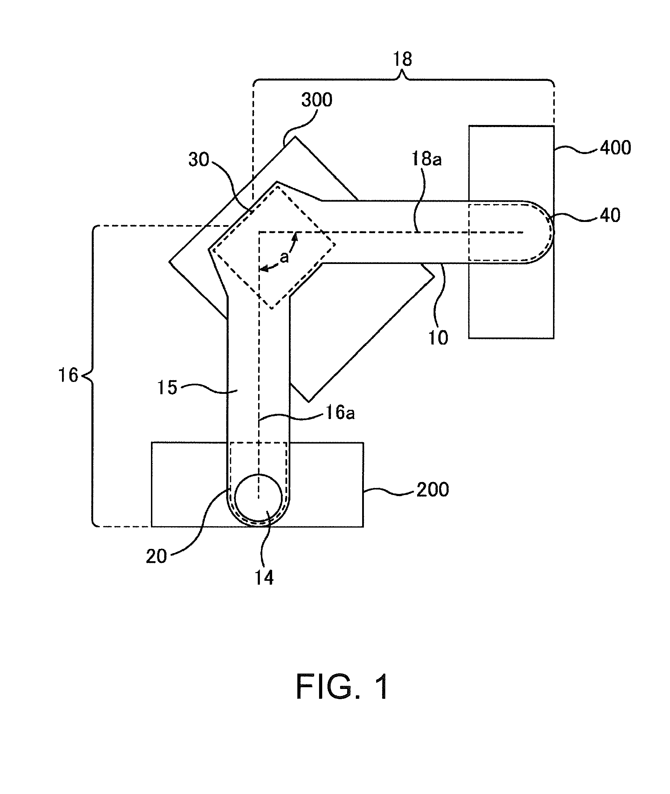

Fig. 1 is a schematic sectional view showing a state in which a substance amplification reaction container 10 is fixed to a first heating section 200, a second heating section 300, and a third heating section 400 in the substance amplification reaction apparatus according to this embodiment.

As shown in Fig. 1, the substance amplification reaction container 10 includes a first channel 16 and a second channel 18. An axis 16a of the first channel 16 and an axis 18a of the second channel 18 form an angle "a". The angle "a" is smaller than 90 degrees.

Liquid 15 and reaction liquid 14 are filled in the substance amplification reaction container 10. The liquid 15 does not mix with the reaction liquid 14, that is, does not dissolve in the reaction liquid 14. Therefore, as shown in Fig. 1, the reaction liquid 14 is retained in the liquid 15 in a state of a droplet. Since the reaction liquid 14 has specific gravity larger than the specific gravity of the liquid 15, the reaction liquid 14 is located in a bottom region in the gravity direction of the substance amplification reaction container 10. As the liquid 15, for example, dimethyl silicone oil or paraffin oil can be used. The reaction liquid 14 is liquid 15 containing a component necessary for reaction. When the reaction is PCR, the reaction liquid 14 includes DNA (target nucleic acid) amplified by the PCR, DNA polymerase necessary for amplifying the DNA, and a primer. For example, when the PCR is performed using oil as the liquid 15, the reaction liquid 14 is preferably an aqueous solution containing the components described above.

The first heating section 200, the second heating section 300, and the third heating section 400 are members that transmit heat generated from a not-shown heater to the substance amplification reaction container 10. In this embodiment, the first heating section 200, the second heating section 300, and the third heating section 400 are heat blocks made of aluminum. Since the heat blocks are made of aluminum, it is possible efficiently heat the substance amplification reaction container 10. Since heating unevenness less easily occurs in the heat blocks, it is possible to realize a highly accurate heat cycle. Since machining is easy, it is possible to accurately mold the heat blocks and improve accuracy of the heating. Therefore, it is possible to realize a more accurate heat cycle.

The first heating section 200, the second heating section 300, and the third heating section 400 respectively include holes. A first region 20, a second region 30, and a third region 40 of the substance amplification reaction container 10 are respectively inserted into the holes of the first heating section 200, the second heating section 300, and the third heating section 400 as shown in Fig. 1. The first region 20 is a region at an end portion of the substance amplification reaction container 10 and an end portion of the first channel 16. The second region 30 is a region located between the first channel 16 and the second channel 18 of the substance amplification reaction container 10. The third region 40 is a region at an end portion of the substance amplification reaction container 10 and an end portion of the second channel 18.

The first region 20 of the substance amplification reaction container 10 is inserted into the hole of the first heating section 200. During heat cycle treatment, the first region 20 of the substance amplification reaction container 10 is heated by the first heating section 200 to raise the temperature of oil in the first region 20 to a first temperature. The first temperature is, for example, 92 to 97°C.

The second region 30 of the substance amplification reaction container 10 is inserted into the hole of the second heating section 300. During the heat cycle treatment, the second region 30 of the substance amplification reaction container 10 is heated by the second heating section 300 to raise the temperature of the oil in the second region 30 to a second temperature. The second temperature is 55 to 72°C.

The third region 40 of the substance amplification reaction container 10 is inserted into the hole of the third heating section 400. During the heat cycle treatment, the third region 40 of the substance amplification reaction container 10 is heated by the third heating section 400 to raise the temperature of the oil in the third region 40 to a third temperature. The third temperature is 65 to 80°C.

The temperatures of the first heating section 200, the second heating section 300, and the third heating section 400 are controlled by a temperature sensor and a control section.

A driving mechanism is provided in the substance amplification reaction apparatus in this embodiment. The driving mechanism is a mechanism for switching the three heating sections to first arrangement, second arrangement, and third arrangement. The driving mechanism includes a motor and a driving shaft. The driving shaft is provided perpendicularly to the second heating section 300. When the motor is operated, the first heating section 200, the second heating section 300, and the third heating section 400 rotate with the driving shaft as an axis of rotation while maintaining a positional relation thereof.

The substance amplification reaction apparatus in this embodiment includes the control section. The control section controls the operation of the driving mechanism to thereby control the first heating section 200, the second heating section 300, and the third heating section 400 to be retained in predetermined arrangement for a predetermined time.

The control section in the substance amplification reaction apparatus may perform electronic control and control all the items. The control section includes a processor such as a CPU and storage devices such as a ROM (Read Only Memory) and a RAM (Random Access Memory) not shown in the figure. Various computer programs, data, and the like for controlling the items are stored in the storage devices. The storage devices include work areas where data being processed, a processing result, and the like of various kinds of processing are temporarily stored.

### 2. Heat cycle treatment performed using the substance amplification reaction apparatus

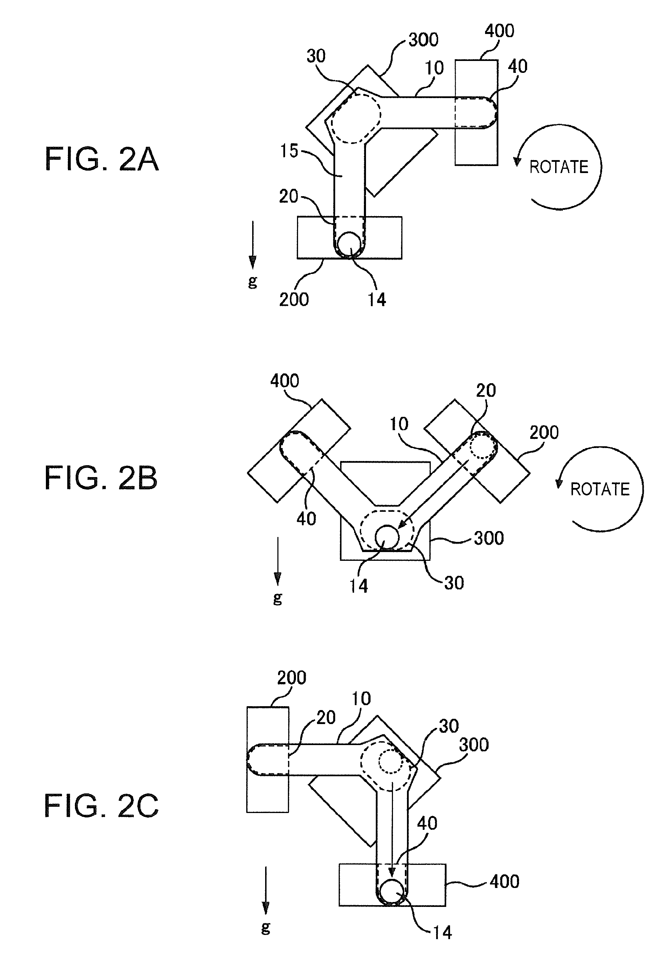

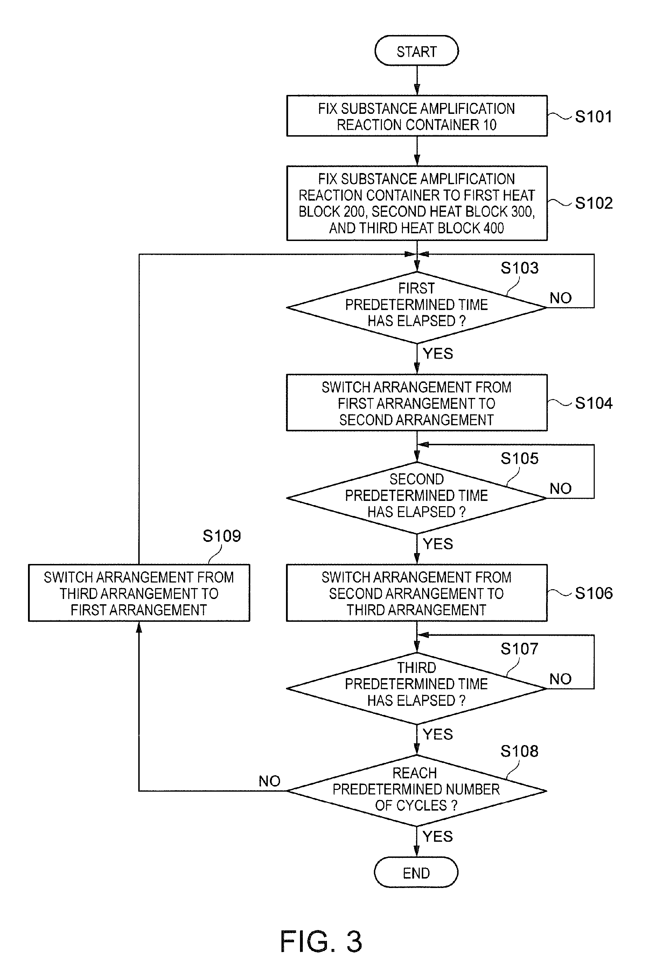

Figs. 2A to 2C are diagrams schematically showing three-step PCR performed using the substance amplification reaction apparatus according to this embodiment. Fig. 2A shows the first arrangement, Fig. 2B shows the second arrangement, and Fig. 2C shows the third arrangement. In Figs. 2A to 2C, a direction of an arrow g (the downward direction in the figures) is a direction in which the gravity acts. Fig. 3 is a flowchart for explaining a procedure of the processing.

Note that, in this specification, concerning two points, "above in the direction in which the gravity acts" and "below in the direction in which the gravity acts" only have to mean that there is a difference between the two points only about the direction in which the gravity acts. A straight line connecting the two points may be parallel to or may not be parallel to the direction in which the gravity acts.

The three-step PCR is a method of repeatedly applying temperature treatment in three stages to reaction liquid to thereby amplify nucleic acid in the reaction liquid. In treatment at high temperature, dissociation of double-stranded DNA is performed. In treatment at low temperature, annealing (a reaction in which the primer combines with single-stranded DNA) and an extension reaction (a reaction in which a complementary chain of DNA is formed starting from the primer) are performed.

In the three-step PCR, the high temperature is temperature between approximately 92°C and 97°C, the low temperature is temperature between approximately 55°C and 72°C, and intermediate temperature is temperature between approximately 65°C and 80°C. The kinds of treatment at the temperatures are performed for a predetermined time. Appropriate times, temperatures, and the number of cycles are different depending on a type and an amount of a reagent in use. Therefore, it is preferable to perform the reaction after determining an appropriate protocol taking into account a type of a reagent and an amount of the reaction liquid 14.

First, the substance amplification reaction container 10 filled with the liquid 15 and the reaction liquid 14 is fixed to the first heating section 200, the second heating section 300, and the third heating section 400 (step S101). The first heating section 200, the second heating section 300, and the third heating section 400 are in contact with the substance amplification reaction container 10 in positions respectively including the first region 20, the second region 30, and the third region 40.

In this embodiment, the arrangement of the first heating section 200, the second heating section 300, and the third heating section 400 in step S101 is the first arrangement. As shown in Fig. 2A, the first arrangement is arrangement in which the first region 20 is below the second region 30 and the third region 40 in the direction in which the gravity acts. In the first arrangement, the first region 20 is located in the bottom of the channel 16 in the direction in which the gravity acts. The reaction liquid 14 having specific gravity larger than the specific gravity of the liquid 15 is located in the first region 20.

In step S102, the substance amplification reaction container 10 is heated by the first heating section 200, the second heating section 300, and the third heating section 400. The first heating section 200, the second heating section 300, and the third heating section 400 heat different regions of the substance amplification reaction container 10 to different temperatures. That is, the first heating section 200 heats the first region 20 to the first temperature, the second heating section 300 heats the second region 30 to the second temperature, and the third heating section 400 heats the third region 40 to the third temperature. Consequently, a temperature gradient in which temperature gradually changes among the first temperature, the second temperature, and the third temperature is formed among the first region 20, the second region 30, and the third region 40. The heat cycle treatment in this embodiment is the three-step PCR. Therefore, it is preferable to set the first temperature to temperature suitable for dissociation of double-stranded DNA, set the second temperature to temperature suitable for annealing, and set the third temperature to temperature suitable for an extension reaction.

The arrangement of the first heating section 200, the second heating section 300, and the third heating section 400 in step S102 is the first arrangement. Therefore, when the substance amplification reaction container 10 is heated in step S102, the reaction liquid 14 is heated to the first temperature. Therefore, in step S102, the reaction liquid 14 is caused to react at the first temperature.

In step S103, it is determined whether the first time has elapsed in the first arrangement. In this embodiment, the determination is performed by the not-shown control section. The first time is time in which the first arrangement is retained. In this embodiment, when step S103 is performed following step S101, that is, when step S103 is performed for the first time, it is determined whether time after the substance amplification reaction apparatus is actuated has reached the first time. In the first arrangement, the reaction liquid 14 is heated to the first temperature. Therefore, the first time is preferably set to time in which the reaction liquid 14 is caused to react at the first temperature in a target reaction. In this embodiment, the first time is preferably set to time necessary for dissociation of double-stranded DNA.

When it is determined in step S103 that the first time has elapsed (YES), the processing proceeds to step S104. When it is determined in step S103 that the first time has not elapsed (NO), step S103 is repeated.

In step S104, the arrangement of the first heating section 200, the second heating section 300, and the third heating section 400 is switched from the first arrangement to the second arrangement by the driving mechanism of the substance amplification reaction apparatus. The second arrangement is arrangement in which the second region 30 is below the first region 20 and the third region 40 in the direction in which the gravity acts (Fig. 2B). In step S104 in this embodiment, the arrangement of the first heating section 200, the second heating section 300, and the third heating section 400 is switched from the state shown in Fig. 2A to the state shown in Fig. 2B.

When the arrangement is switched from the first arrangement to the second arrangement in step S104, the reaction liquid 14 moves from the first region 20 to the second region 30 according to the action of the gravity. When the arrangement of the first heating section 200, the second heating section 300, and the third heating section 400 reaches the second arrangement, step S105 is started. In step S105, it is determined whether the second time has elapsed in the second arrangement. The second time is time in which the first heating section 200 and the second heating section 300 are retained in the second arrangement. In this embodiment, the second region 30 is heated to the second temperature in step S102. Therefore, in step S105 in this embodiment, it is determined whether time after the arrangement reaches the second arrangement has reached the second time. In the second arrangement, since the reaction liquid 14 is retained in the second region 30, the reaction liquid 14 is heated to the second temperature in time in which the first heating section 200 and the second heating section 300 are retained in the second arrangement. Therefore, the second time is preferably set to time in which the reaction liquid 14 is heated to the second temperature in the target reaction. In this embodiment, the second time is preferably set to time necessary for annealing.

When it is determined in step S105 that the second time has elapsed (YES), the processing proceeds to step S106. When it is determined in step S105 that the second time has not elapsed (NO), step S105 is repeated.

In step S106, the arrangement of the first heating section 200, the second heating section 300, and the third heating section 400 is switched from the second arrangement to the third arrangement by the driving mechanism of the substance amplification reaction apparatus. The third arrangement is arrangement in which the third region 40 is below the first region 20 and the second region 30 in the direction in which the gravity acts (Fig. 2C).

When the arrangement is switched from the second arrangement to the third arrangement in step S106, the reaction liquid 14 moves from the second region 30 to the third region 40 according to the action of the gravity. When the arrangement of the first heating section 200, the second heating section 300, and the third heating section 400 reaches the third arrangement, step S107 is started. In step S107, it is determined whether the third time has elapsed in the third arrangement. The third time is time in which the third arrangement is retained. In this embodiment, the third region 40 is heated to the third temperature in step S102. Therefore, in step S107 in this embodiment, it is determined whether time after the arrangement reaches the third arrangement has reached the third time. In the third arrangement, the reaction liquid 14 is retained in the third region 40. Therefore, in time in which the arrangement of the first heating section 200, the second heating section 300, and the third heating section 400 are retained in the third arrangement, the reaction liquid 14 is heated to the third temperature. Therefore, the third time is preferably set to time in which the reaction liquid 14 is heated to the third temperature in the target reaction. In this embodiment, the third time is preferably set to time necessary for an extension reaction.

When it is determined in step S107 that the third time has elapsed (YES), the processing proceeds to step S108. When it is determined in step S107 that the third time has not elapsed (NO), step S107 is repeated.

In this embodiment, the substance amplification reaction container 10 includes a bent section and includes, on both sides of the bent section, the first channel 16 and the second channel 18 in which the reaction liquid 14 moves. The axis 16a of the first channel 16 and the axis 18a of the second channel 18 form an angle smaller than 90 degrees. The first region 20, the second region 30, and the third region 40 can be maintained at the temperatures. Therefore, it is possible to amplify a substance at high speed by moving the reaction liquid 14 in the oil in the substance amplification reaction container 10.

In step S108, it is determined whether the number of times of the heat cycle reaches a predetermined number of cycles. Specifically, it is determined whether the procedure of step S103 to step S107 has been completed a predetermined number of times. In this embodiment, the number of times step S103 to step S107 are completed is determined according to the number of times it is determined "YES" in step S103, step S105, and step S107. When step S103 to step S107 are performed once, the heat cycle is applied to the reaction liquid 14 by one cycle. Therefore, the number of times step S103 to step S107 are performed can be set as the number of cycles of the heat cycle. Therefore, according to step S108, it is possible to determine whether the heat cycle has been applied to the reaction liquid 14 the number of times necessary for the target reaction.

When it is determined in step S108 that the heat cycle has been performed the predetermined number of cycles (YES), the processing is completed (END). When it is determined in step S108 that the heat cycle has not been performed the predetermined number of cycles (NO), the processing shifts to step S109.

In step S109, the arrangement is switched from the third arrangement to the first arrangement. When the arrangement reaches the first arrangement, step S103 is started.

When step S103 is performed following step S109, that is, when step S103 is performed for the second and subsequent times, it is determined whether time after the arrangement reaches the first arrangement has reached the first time.

The invention is not limited to the embodiment explained above and various modifications of the embodiment are possible. For example, the invention includes components substantially the same as the components explained in the embodiment (for example, components having functions, methods, and results same as the functions, the methods, and the results of the components explained in the embodiment or components having objects and effects same as the objects and the effects of the components explained in the embodiment). The invention includes components in which unessential portions of the components explained in the embodiment are replaced. The invention includes components that attain action and effects same as the action and effects of the components explained in the embodiment or components that can attain objects same as the objects of the components explained in the embodiment. The invention includes components obtained by adding publicly-known techniques to the components explained in the embodiment.

## Claims

1. A substance amplification reaction apparatus comprising:
a mounting section mountable with a substance amplification reaction container (10) filled with reaction liquid (14) and liquid (15) that has specific gravity different specific gravity of the reaction liquid (14) and does not mix with the reaction liquid (14) and having a channel in which the reaction liquid (14) moves, the channel including a first channel (16) and a second channel (18), the first channel (16) and the second channel (18) being connected via a bent section, and an axis of the first channel (16) and an axis of the second channel (18) forming an angle smaller than 90 degrees;
a first heating section (200) capable of heating a first region (20), which is a region of an end portion on the first channel side of the channel;
a second heating section (300) capable of heating a second region (30), which is a region of the bent section;
a third heating section (400) capable of heating a third region (40), which is a region at an end portion on the second channel side of the channel;
a driving mechanism configured to switch among a first arrangement, a second arrangement, and a third arrangement in this order; and
a control section configured to control the driving mechanism to maintain the first arrangement for a predetermined time, thereafter maintain the second arrangement for the predetermined time, and thereafter maintain the third arrangement for the predetermined time, wherein
the first arrangement is an arrangement in which the first region (20) is below the second region (30) and the third region (40) in a direction in which the gravity acts,
the second arrangement is an arrangement in which the second region (30) is below the first region (20) and the third region (40) in the direction in which the gravity acts, and
the third arrangement is an arrangement in which the third region (40) is below the first region (20) and the second region (30) in the direction in which the gravity acts.

2. The substance amplification reaction apparatus according to claim 1, wherein
the first heating section (200) includes a first hole and the first hole is capable of housing the first region (20),
the second heating section (300) includes a second hole and the second hole is capable of housing the second region (30), and
the third heating section includes a third hole and the third hole is capable of housing the third region (40).

3. The substance amplification reaction apparatus according to claim 2, wherein the mounting section is configured by the first to third holes.

4. The substance amplification reaction apparatus according to any one of claims 1 to 3, wherein the substance is nucleic acid.

5. A method of amplifying a substance using a substance amplification reaction apparatus, the substance amplification reaction apparatus including:
a mounting section mountable with a substance amplification reaction container (10) filled with reaction liquid (14) and liquid (15) that has specific gravity different specific gravity of the reaction liquid (14) and does not mix with the reaction liquid (14) and having a channel in which the reaction liquid (14) moves, the channel including a first channel and a second channel, the first channel and the second channel being connected via a bent section, and an axis of the first channel and an axis of the second channel forming an angle smaller than 90 degrees;
a first heating section (200) capable of heating a first region (20), which is a region of an end portion on the first channel side of the channel;
a second heating section (300) into which a second region (30), which is a region of the bent section, is insertable; and
a third heating section (400) capable of heating a third region (40), which is a region at an end portion on the second channel side of the channel,
the method comprising maintaining, for a predetermined time, a first arrangement in which the first region (20) is below the second region (30) and the third region (40) in a direction in which gravity acts, thereafter, transitioning to a second arrangement in which the second region (30) is below the first region (20) and the third region (40) in the direction in which the gravity acts and maintaining the second arrangement for the predetermined time, and thereafter transitioning to a third arrangement in which the third region (40) is below the first region (20) and the second region (30) in the direction in which the gravity acts and maintaining the third arrangement for the predetermined time.

6. The method according to claim 5, wherein the substance is nucleic acid.
